(19) Europäisches Patentamt / European Patent Office / Office européen des brevets

(11) **EP 4 438 089 A1**

(12) **EUROPEAN PATENT APPLICATION**

(43) Date of publication:
**02.10.2024 Bulletin 2024/40**

(21) Application number: **23165169.6**

(22) Date of filing: **29.03.2023**

(51) International Patent Classification (IPC):
**A61M 16/00** (2006.01)    **A61M 16/10** (2006.01)
**A61M 16/16** (2006.01)

(52) Cooperative Patent Classification (CPC):
**A61M 16/16; A61M 16/0051; A61M 16/026;**
**A61M 16/109;** A61M 16/0066; A61M 16/161;
A61M 2016/0027; A61M 2016/0039; A61M 2205/18;
A61M 2205/3334; A61M 2205/3358;
A61M 2205/3368; A61M 2205/3389;
A61M 2205/3653; A61M 2205/505;    (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **DUINEVELD, Paulus Cornelis**
**5656 AG Eindhoven (NL)**
• **BOONSTRA, Bonne Lambert**
**5656 AG Eindhoven (NL)**

(74) Representative: **Philips Intellectual Property & Standards**
**High Tech Campus 52**
**5656 AG Eindhoven (NL)**

(54) **METHOD TO DETERMINE AN INITIAL WATER VOLUME IN A HUMIDIFIER AND PREDICTING THE WATER VOLUME OVER TIME**

(57)      A humidifier (52) of a heated pass-over type comprises a heater plate (70), water reservoir (62), sensors (86), and a controller (88). The sensors generate signals for conveying humidifier operating status and ambient conditions. The controller is configured to: (i) predict a water mass within the water reservoir based on predetermined heat and water mass transfer functions of a given humidifier design, the sensor signals, and heater plate power; (ii) determine a length of time to evaporate all water of the predicted water mass; and (iii)(a) output a warning signal in response to predicted water mass being insufficient for a remainder of the treatment duration, or (iii)(b) adapt a power setting to change the evaporation rate of the current predicted water mass to ensure that it is sufficient to last for a remainder of the treatment duration and prevent the water reservoir from running dry.

FIG. 2

(52) Cooperative Patent Classification (CPC): (Cont.)
    A61M 2205/52

**Description**

BACKGROUND

The present embodiments relate generally to humidifiers of a heated pass-over type for use in a sleep or respiratory therapy device and more particularly, to a humidifier configured for determining an initial water volume and predicting the water volume over time and a method thereof.

A humidifier is an important component in a sleep or respiratory device. It increases the relative humidity of the air entering a patient airway such that it is comfortable for the patient to inhale.

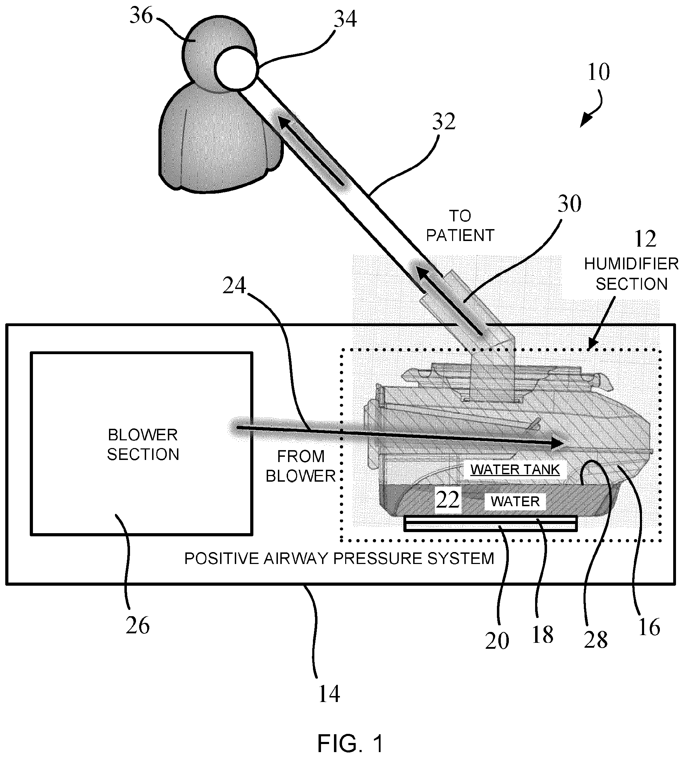

With reference now to Fig. 1, an example of a current configuration 10 of a pass-over type humidifier 12 for use in a positive airway pressure (PAP) respiratory system 14 consists of a water tank 16 that includes a metal plate 18 in contact with a heater plate 20. The water tank 16 is filled with distilled water 22 by the customer. A flow of breathable air 24 from a blower section 26 enters the water tank 16, where the flow of breathable air 24 is heated up by part of the power dissipation of the fan and motor (not shown) of the blower section 26. The air entering the water tank 16 flows over the water surface 28, picking up heat and moisture. The humidified flow of breathable air 30 leaves the humidifier 12 through a tube or patient circuit 32 that connects to a mask or patient interface 34 worn by a patient 36.

The mass flow of moisture to the patient should be controlled as the required mass flow will depend on the environmental conditions, such as ambient temperature and relative humidity, as well as device settings, such as the average pressure and flow rate generated by the fan, as well as patient parameters. Currently, the evaporation rate to the patient is controlled by setting the heater plate temperature. For each new humidifier type, the evaporation rate is determined completely experimentally as function of the heater plate temperature, mask pressure, average air flow rate through the fan, ambient temperature and relative humidity. In the appliance, there is a control algorithm which implements transfer functions (i.e., respectively based upon the experimentally determined evaporation rates) to ensure that the evaporation rate remains at the desired value during operation.

It would thus be desirable to predict an initial water mass, as well as, a time to evaporate all the water contained in the water reservoir or water tank upfront (i.e., at the beginning of a humidification treatment session). In addition, it would be desirable to provide a warning signal to the device user when the water volume would not be sufficient for a full night (i.e., for a full duration of the humidifier treatment session). Moreover, it would be desirable to adjust the evaporation rate during sleep (i.e., during the humidifier treatment session) to prevent that the water reservoir runs dry.

In U.S. patent number 10,220,177, a method is described for predicting the water level in a humidifier water reservoir. The method is based on applying power to a heater plate and then varying the power (i.e., decrease, increase or fully stop) and measuring a rate of change of a temperature. A temperature is measured prior to heating and a temperature is measured at the end of the time period. The ' 177 patent discloses that the particular temperature can be either the water temperature or the heater plate temperature (or other temperatures). In addition, the ' 177 patent discloses that water mass can be determined from a look up table. In particular, the level of water within the chamber is determined using a look table that relates the temperature drop with the water level within the chamber. The relationship between temperature drop and water level is determined experimentally and stored into the memory associated with the controller. The basic relationship between water level and temperature drop is: the larger the water mass or volume the longer it takes for the temperature to drop. A larger volume or mass of the water results in a longer cool down period. Furthermore, the '177 patent mentions an option to be independent of manufacturing variations in, for example, the heater plate heating characteristics. Such an option is with regards to a method where a temperature drop of the heater plate is measured when the heating power is switched-off or reduced, and not a constant powered-on electrical input power for the measurement of temperature drop.

Accordingly, an improved method and apparatus for overcoming the problems in special-use cases in the art is desired.

SUMMARY

According to one aspect, the embodiments of the present disclosure advantageously provide a control algorithm configured to predict an initial water mass, as well as, a time to evaporate all the water contained in the water reservoir or water tank upfront (i.e., at the beginning of a humidification treatment session). In addition, according to another aspect, the embodiments of the present disclosure advantageously provide a warning signal to the device user when the water volume would not be sufficient for a full night (i.e., for a full duration of the humidifier treatment session). Moreover, the embodiments adjust the evaporation rate during the humidifier treatment session to advantageously prevent that the water reservoir runs dry.

The embodiments of the present disclosure simplify a control strategy of a pass-over type humidifier by not changing the power to the heater plate during an initial heating up of the heater plate and water bath, i.e., to keep the heating power constant and preferably at a given maximal steady state power. In addition, the input power to the heater plate can remain constant, independent of a thermal resistance of the heater plate, as will be discussed further herein.

Two features of the embodiments of the present disclosure include a method to determine the initial water mass based on application of constant electrical input power to the heating plate. Determining the initial water mass is based on control algorithms that make use of measuring the heater plate temperature and determining the heater plate temperature first derivative with respect to time. A second feature of the method includes applying a waiting time (i.e., a heater plate power application delay) after the humidifier appliance is switched on for application of power to the heating element or heating plate.

According to one embodiment, a humidifier of a heated pass-over type for use in a sleep or respiratory therapy device that includes a blower section having a blower for supplying a pressurized flow of breathable gas, comprises a heater plate, a water reservoir, one or more sensors, and a controller. The water reservoir is structured to house a volume of water and includes a breathable gas inlet and a humidified breathable gas outlet. The water reservoir further includes at least one surface for contacting the heater plate. The one or more *sensors* are configured to generate output signals conveying information about an operating status of the humidifier and ambient conditions. The controller is configured to: (i) predict *a* water mass of the volume of water contained within the water reservoir at a predetermined time during a humidifier treatment session of a given treatment duration based on predetermined heat and water mass transfer functions of a given design of the humidifier, the output signals conveying information about the operating status of the humidifier and ambient conditions, and on power (e.g., a constant electrical input power) to the heater plate; (ii) determine a length of time needed to evaporate, at a desired (or predetermined) evaporation rate, all water of the predicted water mass; and (iii) at least one of (a) output a warning signal in response to the determined length of time to evaporate all water of the predicted water mass being insufficient for a remainder of the given treatment duration starting from the predetermined time, and (b) adapt a heater plate constant electrical input power setting to change the desired evaporation rate to a new evaporation rate of a current predicted water mass to ensure that the current predicted water mass is sufficient to last for a remainder of the given treatment duration and prevent the water reservoir from running dry prior to an ending of the given treatment duration of the humidifier treatment session.

In another embodiment, the humidifier includes wherein the predetermined time comprises (i) an initial time period at a start of the humidifier treatment session and/or (ii) an intermediate time period of the humidifier treatment session, subsequent to the initial time period. As will be understood further herein, there are two delay times, e.g., a first delay time with respect to determining an initial water temperature and a second delay time, which is actually not a single time, but rather a time interval, which needs to be passed before a water mass determination/estimation can be made. Hence, the time to evaporate all the water of the determined water mass is determined subsequent to the passing of first and second time delays; however, the first and second time delays could also be understood to be a portion of total time needed to evaporate the water. In one embodiment, the initial time period comprises within a time period immediately after switching the humidifier power ON, but not yet applying power (e.g., the constant electrical input power) to the heater plate. According to another embodiment, predicting the water mass does not depend on a thermal contact between the heater plate and a metal plate of the water reservoir (i.e., the metal plate being on or integrated with the surface of the water reservoir for contacting the heater plate). In a further embodiment, the warning signal comprises at least one of an audible alarm, a visual alarm, a tactile alarm, or a combination thereof.

According to another embodiment, the controller is further configured to: (iii) monitor the water mass (i.e., in the water reservoir) by repeating the predicting of the water mass multiple times during the humidifier treatment session, and (iv) determine a corresponding real-time evaporation rate based on a change in the water mass between two or more of the respective multiple times during the humidifier treatment session. In addition, the evaporation rate can be obtained from a transfer function and the water mass as a function of time. In principle, it is possible to use multiple different attempts with slightly different $t_s$ values (as discussed further herein) to calculate the water mass and then take an average. In a further embodiment, the controller is configured to predict the water mass of the volume of water further according to a power control algorithm for humidifying the flow of breathable gas received at the breathable gas inlet of the water reservoir into a flow of humidified breathable gas at the humidified breathable gas outlet of the water reservoir, wherein the power control algorithm includes the transfer function in which a required power input to the heater plate is a function of a desired evaporation rate based upon generated sensor output signals.

According to another embodiment, determining the water mass comprises determining a value of water mass multiple times over an initial period (or over a water mass determination period) of time, and calculating an initial water mass by averaging the multiple determined values of water mass obtained over the water mass determination period. In one embodiment, the operating status of the humidifier includes a heater plate temperature, and the initial period (i.e., water mass determination period) of time comprises a period of time after constant electrical input power is applied to the heater plate up until a time $t_s$, wherein the time $t_s$ corresponds to a time when a condition that (i) a first derivative in the heater plate temperature is less than 50% larger than a quasi-steady state first derivative of the heater plate temperature or (ii) the first derivative in the heater plate temperature is less than 10% larger than the quasi-steady state first derivative of the heater plate temperature has been reached. In addition, determining the length of time needed to evaporate all water of the predicted water mass is based on the initial predicted water mass and an average heater plate temperature gradient during the predetermined time.

In yet another embodiment, the one or more sensors comprise a temperature sensor for measuring a temperature of the heater plate, but no separate temperature sensor for the measuring a temperature of the water in the water reservoir, and wherein predicting the water mass comprises predicting an initial water mass. Predicting the initial water mass comprises: determining an initial water temperature based on the heater plate temperature during a delay period of heater plate power application which occurs immediately subsequent to switching of the humidifier ON but prior to an application of constant electrical input power to the heater plate, measuring and recording the heater plate temperature during the delay period, wherein the delay period expires in response to a first derivative of the heater plate temperature with respect to time during the delay period having obtained a value less than or equal to a minimum first derivative threshold value, applying, upon expiration of the delay period, the constant electrical input power to the heater plate and both (i) measuring and recording the heater plate temperature and (ii) determining at least another first derivative of the heater plate temperature with respect to time.

In addition, predicting the initial water mass further comprises determining a time, based on the measured and recorded heater plate temperature over time, at which (i) the another first derivative in the heater plate temperature is less than 50% larger than a quasi-steady state first derivative of the heater plate temperature or (ii) the another first derivative in the heater plate temperature is less than 10% larger than the quasi-steady state first derivative of the heater plate temperature, has been reached. A heater plate temperature profile is then created that includes sampled heater plate temperatures collected over a profile creation period of time (i) beginning with the application of the constant electrical input power to the heater plate and (ii) ending upon detection of a variation in first derivative of the heater plate temperature to time determined during the profile creation period becoming less than or equal to a minimum first derivative variation threshold amount. A gradient in temperature of the water within the water reservoir is calculated based on the heater plate temperature profile.

The water temperature is estimated based on the initial water temperature and a small addition which is a function of the power and the water mass. The initial water mass is then predicted according to a water mass temperature relationship between (i) the heater plate temperature, (ii) the estimated water temperature, (iii) system parameters for the given humidifier design, and (iv) known humidifier device parameters. The system parameters and humidifier device parameters include water-air interface surface area, heater plate heat loss coefficient, water heat loss coefficient, mass transfer coefficient, heater plate electrical input power, and heat transfer from blower to air. In one embodiment, the correction factor is determined based on (i) a change in heater plate temperature in which the heater plate temperature has increased in a range between 3 to 6 degrees from a temperature of the heater plate when the constant electrical input power to the heater plate is first applied to the heater plate subsequent the heater plate power application delay period, or (ii) an increase depending on the constant electrical input power and the predicted water mass.

According to yet another embodiment, the one or more sensors comprise a temperature sensor for measuring a temperature of the heater plate, and a separate temperature sensor for the measuring a temperature of the water in the water reservoir. In this embodiment, the controller is configured to predict the water mass of the volume of water based on the predetermined heat and water mass transfer functions that include heat and water mass temperature relationships between (i) the heater plate temperature, (ii) the estimated water temperature, (iii) system parameters for the given humidifier design, and (iv) known humidifier device parameters. The system parameters and humidifier device parameters include water-air interface surface area, heater plate heat loss coefficient, water heat loss coefficient, mass transfer coefficient, heater plate electrical input power, and heat transfer from blower to air.

In another embodiment, a gas delivery system for delivering a pressurized flow of humidified breathable gas to a patient via a patient circuit comprises a blower assembly and a humidifier according to an embodiment of the present disclosure. The blower assembly includes a blower adapted to generate the pressurized flow of breathable gas, and a gas flow path including an inlet and an outlet. The humidifier is fluidically coupled between the blower and the patient circuit, wherein the blower assembly further includes at least one of the sensors configured to generate output signals conveying information used in predicting the water mass.

In yet another embodiment, a method for determining water mass in a humidifier of a heated pass-over type for use in humidifying a flow of breathable gas in a sleep or respiratory therapy device having a blower section that includes a blower for supplying a pressurized flow of breathable gas, the method comprising: providing a heater plate; providing a water reservoir structured to house a volume of water, the water reservoir having a breathable gas inlet and a humidified breathable gas outlet, wherein the water reservoir includes at least one surface for contacting the heater plate; and providing one or more sensors configured to generate output signals conveying information about an operating status of the humidifier and ambient conditions.

The method further comprises predicting, via a controller, a water mass of the volume of water contained within the water reservoir at a predetermined time during a humidifier treatment session of a given treatment duration based on predetermined heat and water mass transfer functions of a given design of the humidifier, the output signals conveying information about the operating status of the humidifier and ambient conditions, and on a constant electrical input power to the heater plate; determining, via the controller, a length of time needed to evaporate, at a desired (or predetermined) evaporation rate, all water of the predicted water mass; and controlling, via the controller, at least one of (a) outputting a warning signal in response to the determined length of time to evaporate all water of the predicted water mass being insufficient for a remainder of the given treatment duration starting from the predetermined time, and (b) adapting a heater plate constant electrical input power setting to change the desired evaporation rate to a new evaporation rate of a current predicted water mass to ensure that the current predicted water mass is sufficient to last for a remainder of the given treatment duration and prevent the water reservoir from running dry prior to an ending of the given treatment duration of the humidifier treatment session.

In one embodiment, the one or more sensors comprise a temperature sensor for measuring a temperature of the heater plate, but no separate temperature sensor for the measuring a temperature of the water in the water reservoir. In addition, predicting, via the controller, includes predicting an initial water mass that comprises: (a) determining an initial water temperature based on the heater plate temperature during a delay period of heater plate power application which occurs immediately subsequent to switching of the humidifier ON but prior to an application of constant electrical input power to the heater plate, (b) measuring and recording the heater plate temperature during the delay period, wherein the delay period expires in response to a first derivative of the heater plate temperature with respect to time during the delay period having obtained a value less than or equal to a minimum first derivative threshold value, (c) applying, upon expiration of the delay period, the constant electrical input power to the heater plate and both (c)(i) measuring and recording the heater plate temperature and (c)(ii) determining at least another first derivative of the heater plate temperature with respect to time, (d) determining a time, based on the measured and recorded heater plate temperature over time, at which (d)(i) the another first derivative in the heater plate temperature is less than 50% larger than a quasi-steady state first derivative of the heater plate temperature (i.e., this is a simpler method, whereby the effect of water temperature is relatively small) or (d)(ii) the another first derivative in the heater plate temperature is less than 10% larger than the quasi-steady state first derivative of the heater plate temperature, has been reached, (e) creating a heater plate temperature profile that includes sampled heater plate temperatures collected over a profile creation period of time (e)(i) beginning with the application of the constant electrical input power to the heater plate and (e)(ii) ending upon detection of a variation in first derivative of the heater plate temperature to time determined during the profile creation period becoming less than or equal to a minimum first derivative variation threshold amount, (f) calculating a gradient in temperature of the water within the water reservoir based on the heater plate temperature profile, (g) estimating the water temperature based on the initial water temperature and a small addition which is a function of the power and the water mass, and (h) predicting the initial water mass according to a water mass temperature relationship between (h)(i) the heater plate temperature, (h)(ii) the estimated water temperature, (h)(iii) system parameters for the given humidifier design, and (h)(iv) known humidifier device parameters. In one embodiment, the system parameters and humidifier device parameters include water-air interface surface area, heater plate heat loss coefficient, water heat loss coefficient, mass transfer coefficient, heater plate electrical input power, and heat transfer from blower to air.

In another embodiment, the method further comprises predicting, via the controller, the water mass of the volume of water based on the predetermined heat and water mass transfer functions that include heat and water mass temperature relationships between (i) the heater plate temperature, (ii) the water temperature, (iii) system parameters for the given humidifier design, and (iv) known humidifier device parameters.

Still further advantages and benefits will become apparent to those of ordinary skill in the art upon reading and understanding the following detailed description.

BRIEF DESCRIPTION OF THE DRAWINGS

The embodiments of the present disclosure may take form in various components and arrangements of components, and in various steps and arrangements of steps. Accordingly, the drawings are for purposes of illustrating the various embodiments and are not to be construed as limiting the embodiments. In the drawing figures, like reference numerals refer to like elements. In addition, it is to be noted that the figures may not be drawn to scale.

Fig. 1 is a simplified block diagram view of a positive airway pressure system including a humidifier of a heated pass-over type for delivering humidified breathing gas to a user;

Fig. 2 is a block diagram view of a positive airway pressure system including a humidifier of a heated pass-over type for delivering humidified breathing gas to a user according to an embodiment of the present disclosure;

Fig. 3 is a table of system parameters of a humidifier of a given design according to an embodiment of the present disclosure;

Figs. 4A and 4B comprise plots of heater plate and water bath temperatures for two cases illustrating a change in temperature of the heater plate and water bath when no power is applied to the heater plate, further with a thermal heater plate resistance $R_{th}$=0.75 K/W;

Fig. 5 is plot of recorded parameters in a detailed example of a measurement run at one setting of a DOE according to an embodiment of the present disclosure;

Figs. 6A-6C comprise plots of heater plate and water bath temperatures based on calculated results for the run of the DOE of Fig. 5, with $R_{th}$=0.75, which include a) temperature profiles for time to reach equilibrium, b) zoomed in view, and c) temperature derivatives according to an embodiment of the present disclosure;

Fig. 7 is a plot of temperature difference between heater plate and water for the run of the DOE of Fig. 5 according to an embodiment of the present disclosure; and

Figs. 8A-8B comprise a simulation of the run of the DOE of Fig. 5, but now with a 10 °C larger water temperature, where a) is a temperature profile of heater plate and water bath for time to reach equilibrium, and b) a plot of temperature derivatives.

## DETAILED DESCRIPTION

The embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which the embodiments of the present may be practiced and to further enable those of skill in the art to practice the same. Accordingly, the examples herein should not be construed as limiting the scope of the embodiments of the present disclosure, which is defined solely by the appended claims and applicable law.

It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting in scope of the embodiments as claimed. It must be noted that as used herein and in the appended claims, the singular forms "a," "an," and "the" include plural reference unless the context clearly dictates otherwise.

Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein can be used in the practice or testing of the embodiments.

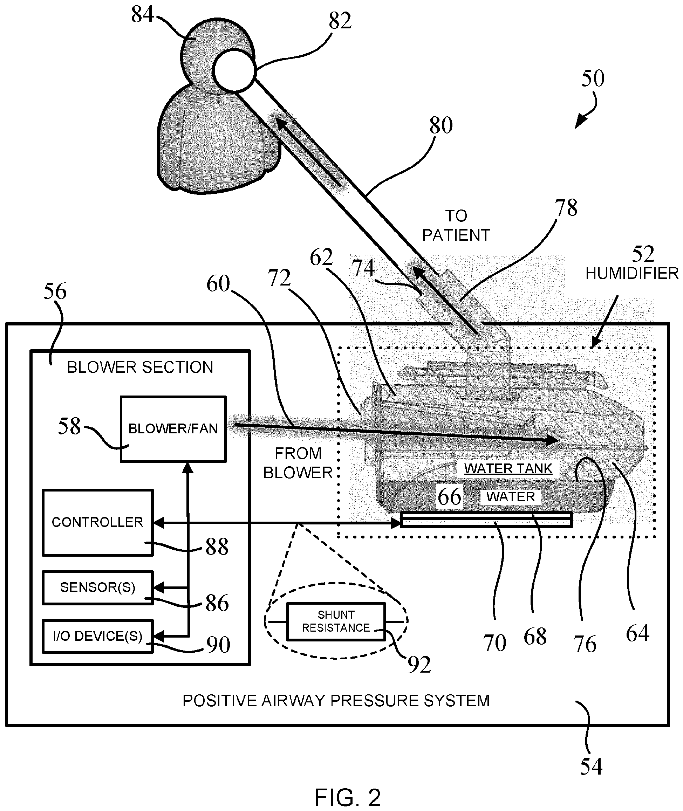

Turning now to Fig. 2, there is shown a configuration 50 of a pass-over type humidifier 52 of a heated pass-over type for use in a sleep and respiratory therapy device or positive airway pressure (PAP) respiratory system 54 according to an embodiment of the present disclosure. The configuration comprises a blower section 56 having a blower 58 for supplying a pressurized flow of breathable gas 60. The humidifier 52 comprises a water reservoir 62 having a water tank 64 structured to house a volume of water 66. The water reservoir 62 further comprises a metal plate 68 on at least one surface of the water reservoir and/or water tank and is configured for contacting with a heater plate 70. The heater plate 70 can also be configured for receiving power from a power source to extend the embodiments to other heating methods/sources such as induction heating. According to another embodiment, the heater plate can comprise (i) an electric heater plate (i.e., thermal heating method), (ii) an inductive heating coil (i.e., inductive heating method), or combination thereof. The water reservoir 62 further includes a breathable gas inlet 72 and a humidified breathable gas outlet 74.

In one embodiment, the water tank 64 comprises a removable water tank which can be removed from the water reservoir 62, via a suitable opening and closing mechanism (not shown) and filled with a volume of distilled water by the user. The filled water tank 64 is then re-inserted into the water reservoir 62. A pressurized flow of breathable gas 60 from the blower section 56 is input to the humidifier 52, via the breathable gas inlet 72, where the flow of breathable gas 60 is heated up by part of the power dissipation of the fan and blower motor 58 of the blower section 56. The air entering the water reservoir 62 flows over the water surface 76, picking up heat and moisture. The humidified flow of breathable air 78 leaves the humidifier 52 through humidified breathable gas outlet 74 and a tube or patient circuit 80 that connects to a mask or patient interface 82 worn by a patient 84.

Humidifier 52 further comprises one or more sensors 86 configured to generate output signals conveying information that indicates one or more device parameters selected from the group consisting of heater plate temperature, an average pressure of the breathable gas, and an average flow rate of the breathable gas, and other parameters that comprise an ambient temperature, a relative humidity, and ambient pressure. In one embodiment, the one or more sensors 86 may further include a water bath temperature sensor. In addition, a controller 88 is configured to control an evaporation rate of the volume of water 66 housed in the water reservoir 62 with an electrical power input control according to a power control algorithm for humidifying the flow of breathable gas 60 received at the breathable gas inlet 72 of the water reservoir 62 into a flow of humidified breathable gas 78 at the humidified breathable gas outlet 74 of the water reservoir 62.

In one embodiment, controller 88 comprises one or more of a microprocessor, microcontroller, field programmable gate array (FPGA), integrated circuit, discrete analog or digital circuit components, hardware, software, firmware, or any combination thereof, for performing various functions as discussed herein, further according to the requirements of a given humidifier device implementation and/or application. Controller 88 can further comprise one or more various modules configured to implement a given functionality.

According to one embodiment, the controller 88 is configured to control an evaporation rate of the volume of water housed in the water reservoir with a power control according to a power control algorithm for humidifying the flow of breathable gas received at the breathable gas inlet of the water reservoir into a flow of humidified breathable gas at the humidified breathable gas outlet of the water reservoir. The power control algorithm includes a transfer function in which a required constant electrical power input to the heater plate is a function of a desired evaporation rate based upon generated sensor output signals.

In one embodiment, the controller implements a power control algorithm that includes a linearized transfer function in which a required constant electrical power input to the heater plate 70 is a function of a desired evaporation rate based upon predetermined device parameters. The linearized transfer function is specific to the blower 58 supplying the flow of breathable gas 60 to the breathable gas inlet 72 of the water reservoir 62, as discussed further herein. In one embodiment, the required electrical power input to the heater plate 70 comprises an electrical power, PWM% in units of %, controlled by pulse width modulation (PWM). In another embodiment, the required power input comprises an electric power input to the heater plate that is operable as a control parameter to regulate the evaporation rate, as will be discussed further herein. In other words, according to the embodiments of the present disclosure, power to the heater plate is actively used to control the evaporation rate. The evaporation rate can further be based on one or more of the operating pressure and blower motor type.

With reference still to Fig. 2, there is shown one or more input/output device(s), collectively indicated by reference numeral 90, which is representative of any number of suitable I/O devices for inputting or outputting one or more of an audible, visual, and/or tactile input or output, according to the requirements of a given humidifier implementation, further as discussed herein. For example, input/output device(s) 90 can comprise one or more of an input/output device, user interface, tactile output device, touch screen, optical display, microphone, keypad, keyboard, pointing device, image capture device, video camera, audio output device, and any combination thereof, according to the requirements of a given humidifier system implementation.

As illustrated in Fig. 2, controller 88, sensor(s) 86 and I/O devices 90 are illustrated as being located within the blower section 56. However, the controller 88, sensor(s) 86 and I/O devices 90 can also be located within the humidifier section 52. Still further, the controller 88, sensor(s) 86 and I/O devices 90 can be distributed between both the blower section 56 and the humidifier section 52, according to the requirements of a given humidifier, blower, and/or positive airway pressure system implementation.

In one embodiment, the electrical power to the heater plate 70 can be measured by using a shunt resistance in case there is too much variation of the electrical resistance of the heater plate (i.e., due to different heater plate manufacturers, manufacturing processes and/or tolerances). In particular, a shunt resistance 92 can be placed in series with power to the heater plate 70. The controller 88 is configured to obtain a measurement of electrical power delivered to the heater plate via measuring a voltage drop across the shunt resistance, determining electrical current to the heater plate using the measured voltage drop across the shunt resistance and voltage, $V_{hp}$, across the heater plate.

As will be discussed further herein, there are at least two methods of determining an initial water mass contained in a humidifier of a given design. In a first method, in which the humidifier is equipped with a temperature sensor for the heater plate, but no separate temperature sensor for the water bath, the method comprises a number of steps which will be explained herein below. A first step includes determining, during a heater plate power application delay period that occurs immediately subsequent to switching of the humidifier ON, the initial water temperature based on the heater plate temperature by waiting a sufficient time before an application of power (e.g., constant electrical input power) is applied to the heater plate. The sufficient waiting time is until the heater plate temperature first derivative with respect to time has reached a sufficiently low value. In other words, determining the initial water temperature is based on measuring/sampling the heater plate temperature during the heater plate power application delay period. The delay period expires in response to the heater plate temperature first derivative with respect to time during the delay period reaching the sufficiently low value (i.e., a value less than or equal to a minimum first derivative threshold value).

A second step includes applying, upon expiration of the delay period, a constant electrical input power to the heater plate and both (i) measuring the heater plate temperature (i.e., sampling and recording) and (ii) determining at least the heater plate temperature first derivative to time. A third step includes determining the time, based on the sampled heater plate temperature over time, at which the variation in first derivative of heater plate temperature is sufficiently small. In other words, the third step includes determining a time when (i) the first derivative to time in the heater plate temperature is less than 50% larger than the quasi-steady state first derivative of the heater plate temperature, and more preferably less than 10% larger than the quasi-steady state first derivative. This time is referred to herein as $t_s$ (or $t_{steady}$).

A fourth step comprises determining a time $t_b$ (or beginning time of an interval) where the first derivative in a gradient in the heater plate temperature (also referred to herein as heater plate temperature gradient or heater plate gradient) is in the range of 1.5-10 times, and more preferably in the range of 3 to 5 times, the quasi-steady state heater plate temperature gradient. A fifth step includes determining a time $t_e$ (or end time of the interval) that corresponds to a moment which is a similar time away from the quasi-steady state heater plate gradient time $t_s$ as the beginning time $t_b$, which fulfils the expression of $t_e \approx 2t_s - t_b$. In other words, the time distance between $t_e$ and $t_s$ is equal to the time distance between $t_s$ and $t_b$. A sixth step comprises determining an average heater plate temperature gradient according to the

expression $\dfrac{1}{t_e - t_b} \int_{t_b}^{t_e} \dfrac{\mathrm{d}T_{hp}}{\mathrm{d}t}\,\mathrm{d}t$ .

A seventh step comprises estimating the water temperature (over the interval of time $t_b$ to $t_e$) based on the initial water temperature and a small addition which is a function of the constant electrical input power and the water mass, as will be discussed further herein.

In a second method, in which the humidifier is equipped with both a temperature sensor for the heater plate and a separate temperature sensor for the water bath, the second method skips the previous steps of the first method, and both methods implement the following steps which will be explained herein below.

A final step in both methods comprises determining the water mass (at a given time) from (i) the estimated water temperature (or measured water temperature in the case of a separate temperature sensor being provided for the water bath), (ii) heating plate temperature, (iii) electrical power and (iv) the steady state relations for the heat and mass loss relations for the given design of the humidifier. In addition, depending on the humidifier device settings (e.g., level of humidification, duration of humidification treatment, etc.), both methods further include providing a warning signal to the user or adapting the heating strategy of the humidifier such that the water volume (i.e., the predicted water mass in the water reservoir or tank) is sufficient for a full day (i.e., sufficient for a full duration of the humidifier treatment session).

Turning now to a more detailed discussion of the first method, basic heat and water mass transfer equations for a given humidifier design are as follows:

$$C_{hp} \cdot \frac{dT_{hp}}{dt} = \frac{V_{hp}^2}{R_{hp}} \cdot \frac{PWM\%}{100\%} - \frac{T_{hp} - T_w}{R_{th}(T_{hp}, T_w, A_{hp}, R_c, \ldots)} - \alpha_{hp_{loss}} \cdot \left(T_{hp} - T_{atm}\right)$$

(Eq. 1)

$$m_w \cdot c_w \cdot \frac{dT_w}{dt} = \frac{T_{hp} - T_w}{R_{th}(T_{hp}, T_w, A_{hp}, R_c, \ldots)} - \alpha_{w_{loss}} \cdot (T_w - T_{atm}) - h(Q, m_w) \cdot A_s(m_w) \cdot \left(T_w - T_{hum_{in}}\right) - \dot{m}_w \cdot L_h$$

(Eq. 2)

The mass transfer of water will change the air properties, which can be written as:

$$\dot{m}_w = k(Q, m_w) \cdot A_s(m_w) \cdot M_w \cdot \left(\frac{p_{v,s}(T_w)}{R_u \cdot T_w} - \frac{RH_{atm} \cdot p_{v,s}(T_{atm})}{R_u \cdot T_{atm}}\right)$$

(Eq. 3)

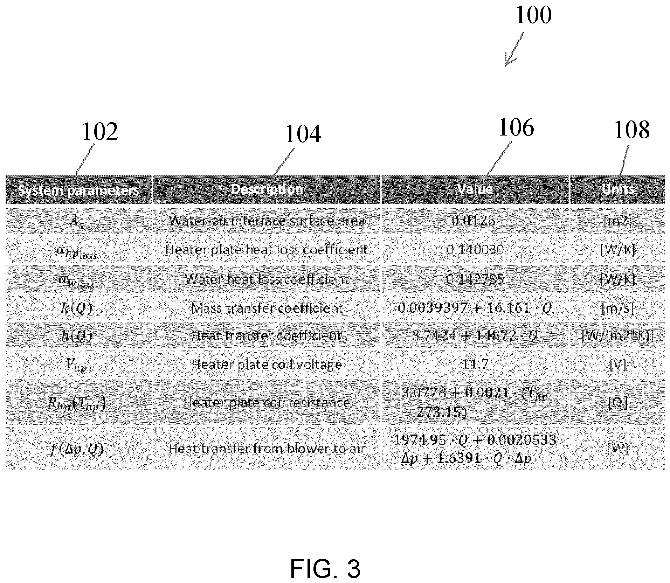

In the three equations (i.e., Eq. 1, 2 and 3), there are a number of parameters, as will be explained. First there are the system parameters, i.e., these are parameters that depend on the humidifier design used. Referring now to Fig. 3, a table 100 of the system parameters 102, descriptions 104, parameter values 106 and units 108 for a given humidifier design according to an embodiment of the present disclosure is shown. In particular, the system parameters 102 include: water-air interface surface area ($A_s$) in units of [m$^2$]; heater plate heat loss coefficient ($\alpha_{hp\ loss}$) in units of [W/K]; water heat loss coefficient ($\alpha_{w\ loss}$) in units of [W/K]; mass transfer coefficient $k(Q)$ in units of [m/s]; and heat transfer coefficient $h(Q)$ in units of [W/(m$^2\cdot$K)]. In addition, heater plate coil electrical resistance ($R_{hp}$) in units of [Ω] is a function of heater plate temperature ($T_{hp}$) in units of [K]. Lastly, $f(\Delta p,\ Q)$ is the heat transferred from the blower fan to the air passing through the fan in units of [W], which is a function of the average pressure and the average flow rate generated by the blower fan. The parameter $f(\Delta p,\ Q)$ is a system parameter that only depends on the air path construction and the fan type being used. The table 100 of system parameters was determined from a design of experiments DOE for the given humidifier design. This included a number of measurement runs for experimental results, based on the three (3) equations (Eq. 1-3), to determine six (6) unknown system variables where the total error has been minimized.

In particular, Equation (1) describes the temperature change of the heater plate which is due to the electrical power to the heating plate (i.e., the first term on the right), the power transmitted to the water bath (i.e., the second term on the right) and the losses to the environment (i.e., the third term on the right). In Equation (1), $C_{hp}$ is the thermal capacity of the heater plate, $T_{hp}$ the temperature of the heater plate, $V_{hp}$ the heater plate coil voltage, $R_{hp}$ the heater plate coil resistance, PWM% the PWM duty cycle of heater plate coil in units of [%], $T_w$ the temperature of the water, $R_{th}$ the thermal resistance of the heater plate in units of K/W (Kelvin per Watt), $A_{hp}$ the surface area of the heater plate, and $T_{atm}$ the atmospheric pressure.

Equation (2) describes the change in the water bath temperature which is, due to the large free convection which is approximately equal to a constant at any given time. This change in the water bath temperature is due to the power transmitted by the heater plate (i.e., first term to the right), the losses to the environment (i.e., second term to the right), the heat transfer to the air (i.e., third term to the right) and the evaporation of water (i.e., the fourth term $(\dot{m}_w \cdot L_h)$). In Equation (2), $m_w$ is the water mass; $c_w$ the specific heat of water; $\alpha_{w\,loss}$ the water heat loss coefficient; $\dot{m}_w$ the water evaporation; and $L_h$ the latent heat of evaporation water.

In equation (3) the water evaporation $(\dot{m}_w)$ is worked out in detail, where $k(Q, m_w)$ is the mass transfer coefficient in units of [m/s]; $A_s(m_w)$ the water-air interface surface area in units of [m2]; $M_w$ the molar mass of water in units of [kg/mol]; $R_u$ the universal gas constant in units of [J/(mol·K)]; $(p_{v,s}(T_w))$ and $(p_{v,s}(T_{atm}))$ the saturated vapor pressure in units of [Pa]; $RH_{atm}$ the ambient relative humidity; $T_w$ the temperature of the water; and $T_{atm}$ the ambient temperature.

In the simplest, most cost-effective embodiment, the humidifier includes a temperature sensor in the heater plate (and no separate temperature sensor in the water bath). Thus, the water bath temperature is therefore unknown, as well as the water mass $m_w$. There are six system parameters, as identified in Table 1 (Fig. 3), that are determined up front for each humidifier design from several experiments for a given humidifier design, as is explained in detail in co-pending patent application, US Serial No. 63/357,311, filed June 30, 2022 (Attorney Docket 2022PF00428), incorporated herein by reference in its entirety. In addition, it has been determined that parameters $h$ and $k$ are only a function of the average air flow $Q$.

Now the aim is to determine the water mass at constant electrical input power (i.e., constant power to the heater plate). Therefore, the method begins with determining the initial water temperature. Determining the initial water temperature is done during an initial power delay to the heating plate by waiting a short time to switch ON the electrical heating power (i.e., the constant power to the heater plate) after the humidifier appliance or device has been switched ON. During this initial heater plate power delay waiting time (or heater plate power application delay period), the temperature of the heater plate is recorded (e.g., sampled at a predetermined sampling rate and recorded). The sampled heater plate temperatures are used to create a heater plate temperature profile of sampled temperatures for the heater plate power application delay period. In other words, the temperature profile refers to a collection of heater plate temperatures measured periodically during the heater plate power application delay period or interval.

There are three (3) different possibilities for the temperature profile. The temperature profile is either indicative of (i) constant heater plate temperature, (ii) the heater plate temperature drops, or (iii) the heater plate temperature increases. In one instance, the temperature profile remains constant over the duration of the heater plate power application delay period, which is indicative of the water bath and heater plate having a similar temperature, e.g., ambient temperature. Hence, in this first instance, a constant heater plate temperature will be recorded (i.e., since both the heater plate and water are at ambient temperature). In a second instance, the heater plate temperature can drop in response to the water tank having just been filled with water that is colder than ambient temperature and, soon thereafter, the water tank being installed within the humidifier upon the heater plate. In a third instance, the heater plate temperature may also increase due to a water temperature being warmer. That is, the water tank having been filled with water that is warmer than the ambient temperature and, soon thereafter, gets installed within the humidifier upon the heater plate will influence the heater plate temperature to increase.

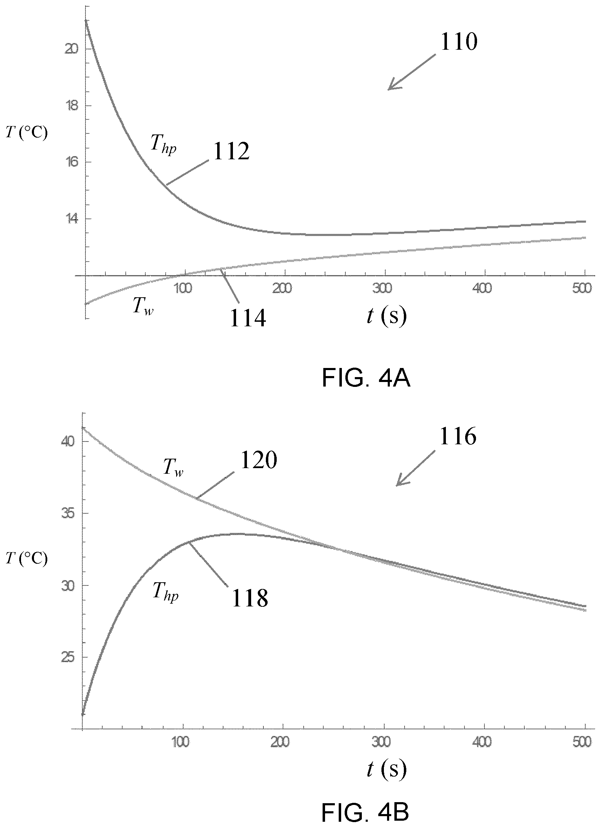

Turning now to Figs. 4A and 4B, plots 110 and 116, respectively, of heater plate and water bath temperatures for two cases are shown which illustrate a change in temperature of the heater plate and water bath when no power has yet been applied to the heater plate, further with a thermal heater plate resistance $R_{th}$=0.75 K/W. In particular, the plots are numerical solution plots of heater plate and water bath temperatures for two cases in which (i) the water bath temperature 114 is initially cooler than the heater plate temperature 112, and (ii) the water bath temperature 120 is initially warmer than the heater plate temperature 118. In the first case (Fig. 4A), the heater plate temperature 112 initially drops or decreases, and in the second case (Fig. 4B), the heater plate temperature 118 initially increases for the given humidifier design. In both cases, the heater plate temperature is almost equal to the water bath temperature within approximately 100-200 seconds.

In addition, the plots in Fig. 4 are numerical solutions of two cases (i.e., one case where the heater plate temperature drops, and another case where the heater temperature increases) for a realistic setting with a humidifier of specific design according to an embodiment of the present disclosure. It is noted that within approximately 100-200 seconds from time t=0 (i.e., when the humidifier is powered ON, but power to the heater plate has not yet been applied), the heater plate temperature $T_{hp}$ is almost equal to the water bath temperature $T_w$. Based on the plots of Fig. 4A (water bath is initially cooler than the heater plate, and Fig. 4B (water bath is initially warmer than the heater plate), a criterion can be applied in case the heater plate temperature changes significantly (e.g., by applying a significant temperature change threshold test to determine whether the heater plate temperature changes beyond a threshold amount during an initial period of time from humidifier power ON) such that the second derivative with respect to time of the heater plate temperature becomes close to zero, or the first derivative with respect to time of the heater plate temperature has just (e.g., preferably after 10 to 50 s) changed sign (e.g., preferably after 5 to 80 s). In any event, the intention is to make use of the heater plate temperature gradient to determine a sufficient waiting time, such that the heater plate temperature is substantially equal to the water plate temperature.

To find an expression for the water mass, we take the derivative of Equation (1) and find an expression for the change in the water temperature as a function of (i) the first and second derivative of the heater plate temperature and (ii) the heater plate loss coefficient as well as (iii) the thermal heater plate resistance $R_{th}$.

$$\frac{dT_w}{dt} = R_{th}(T_{hp}, T_w, A_{hp}, R_c, \dots)C_{hp} \cdot \frac{d^2 T_{hp}}{dt^2} + \frac{dT_{hp}}{dt}\left(1 + \alpha_{hp_{loss}} \cdot R_{th}(T_{hp}, T_w, A_{hp}, R_c, \dots)\right)$$

$$\text{(Eq. 4)}$$

This thermal heater plate resistance $R_{th}$ can be obtained from the steady state solution of a previous measurement and follows from either Equation (1) or (2) where the steady state mass transfer and water temperature are known, as well as all system parameters and environmental parameters. From both Equation (1) or (2), we can determine thermal heater plate resistance $R_{th}$ and as a value we can either select (i) the average value or (ii) the value that belongs to Equation (1), or even Equation (2).

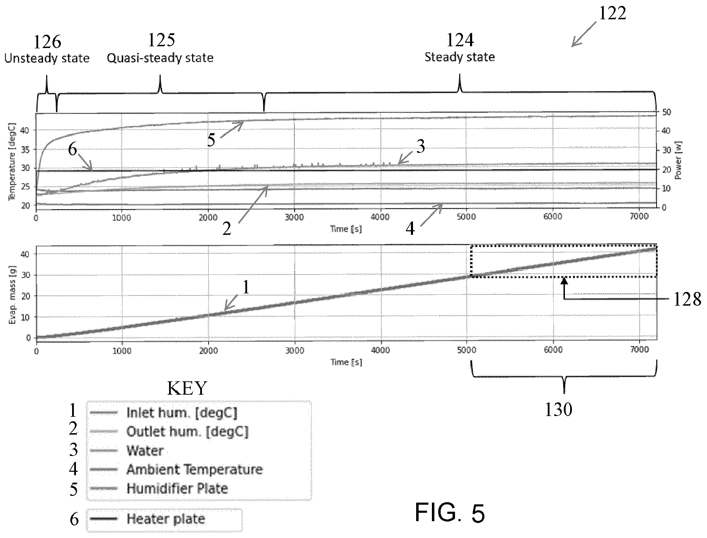

With reference now to Fig. 5, there is shown a plot of recorded parameters in a detailed example of a measurement run at one setting of a DOE according to an embodiment of the present disclosure. The plot of recorded parameters comprises experimental results for the temperature change of both (i) the water bath (wherein the water bath temperature was measured with a separate thermocouple in the water) as well as (ii) the heater plate temperature to explain the strategy which is selected to calculate the change in water bath temperature. In particular, Fig. 5 is plot 122 of recorded parameters in a detailed example of a measurement run at one setting of a DOE. All relevant parameters were recorded just after steady state 124 is reached, which takes some time, as shown in Fig. 5. Note that we see an initially rapid increase of the heater plate temperature (e.g., within the first 1000 seconds) in the 'unsteady state' 126, while the water temperature slowly increases (e.g., within the first 3000 seconds) through quasi-steady state to the steady state values. The evaporation rate is calculated by performing linear regression on the evaporated mass, during steady state 128. Quasi-steady state 125 occurs subsequent the unsteady state 126 and prior to steady state 124 in Fig. 5. In other words, unsteady state 126 is characterized by a rapidly varying first derivative of the heater plate temperature. The quasi-steady state 125 is characterized by an almost constant first derivative. Lastly, steady state 124 is characterized by the heater plate temperature being almost constant and therefore the first derivative almost zero. In one embodiment, steady state results 128 are determined by averaging over the last 2000 seconds, as indicated by reference numeral 130.

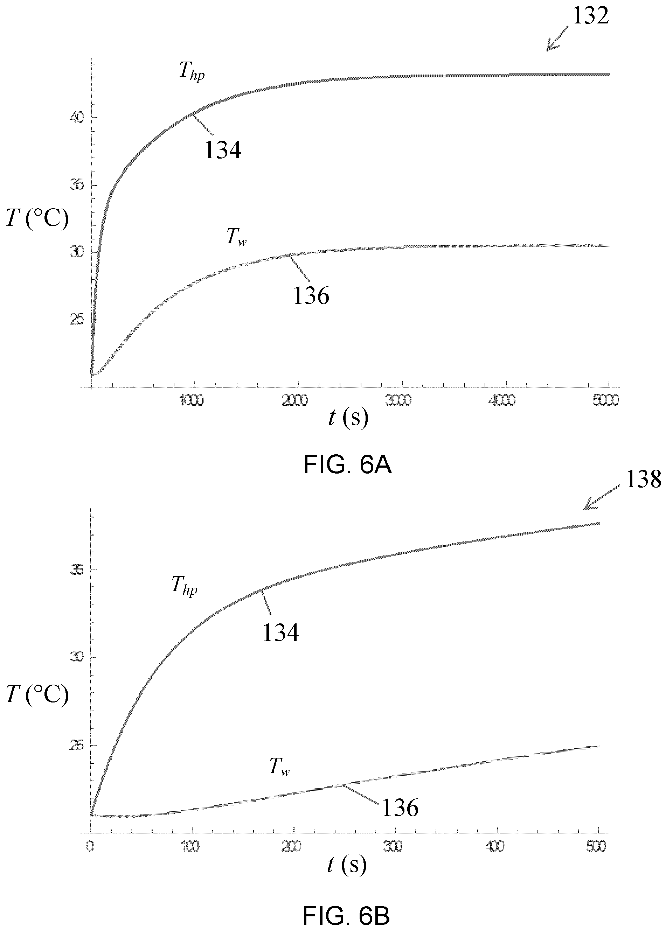

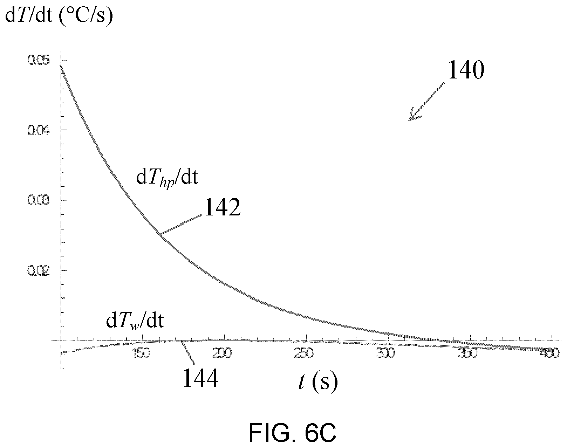

In Fig. 6, calculated results for a given run DOE are shown which are based on the model Equations (1) - (3), with thermal heater plate resistance $R_{th}$=0.75 K/W. In Fig. 6(A), a temperature profile 132 for time to reach equilibrium is shown for the heater plate temperature $T_{hp}$ 134 and the water bath temperature $T_w$ 136. Fig. 6(B) is a zoomed in portion 138 of Fig. 6(A) showing the heater plate temperature $T_{hp}$ 134 and the water bath temperature $T_w$ 136 from t=0 to t=500 seconds. Fig. 6(C) illustrates temperature derivatives for the heater plate temperature $T_{hp}$ and the water bath temperature $T_w$ from t=0 to t=400 seconds. In addition, with respect to the calculated results shown in Figs. 6A-C, the inlet temperature to the humidifier ($T_{hum_{in}}$) was calculated according to Equation (5), with $f(\Delta p, Q)$ (i.e., the heat transfer from blower to air) taken from Table 1 (i.e., shown in Fig. 3).

$$T_{hum_{in}} = T_{atm} + \frac{f(\Delta p, Q)}{\rho_{air_{atm}}(p_{atm}, T_{atm}, RH_{atm}) \cdot Q \cdot c_p}$$

$$\text{(Eq. 5)}$$

In Equation (5), $T_{atm}$ is the ambient temperature, $f(\Delta p, Q)$ the heat transfer from blower to air; $\rho_{air_{atm}}(p_{atm}, T_{atm}, RH_{atm})$ the ambient air density in units of [kg/m³]; $Q$ the average air flow rate; and $c_p$ the specific heat of air (i.e., 1008 J/(Kg °C) around room temperature). As previously noted, $f(\Delta p, Q)$ is the power transferred from the blower fan to the air passing through the blower fan, which is a function of the average pressure and the average flow rate generated by the fan. The parameter $f(\Delta p, Q)$ is a system parameter that only depends on the air path construction and the blower fan type being used.

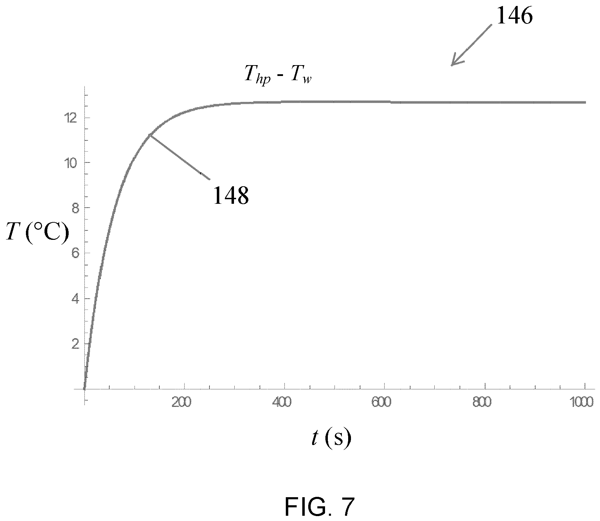

Note that in Fig. 6(C) the first derivative has also been calculated and indeed it can be seen that the derivative of both heater plate and water become close to each other after some time as the second derivative of the heater plate is small as well as the thermal resistance and the heater plate loss coefficient, which is what we also expect from Equation (4). This is also reflected in Fig. 7, which illustrates a plot of the temperature difference between the heater plate and water bath for the given run DOE. Although it takes a long time until equilibrium is obtained, the difference between the heater plate and water bath temperatures becomes constant after approximately 250-300s, of course in line with Fig. 6(C). This is due to the small thermal mass of the heater plate compared to the thermal mass of the water tank (i.e., when the water tank is completely filled with water). With respect to actual experiments, the difference between the heater plate and the water temperatures is not exactly constant, but substantially constant.

The temperature of the water at the time $t_b$ can be determined via a simple method as the initial water temperature (obtained with the heater plate sensor) and an increase. The increase can be selected from either a constant increase of the water temperature (in the range of 3-6 degrees), or an increase depending on the power and water mass. In other words, the water mass is varying only slightly when the water temperature is varying with several degrees, and thus the water mass is not very sensitive on the exact water temperature.

Now that the water bath temperature is determined via estimation (or via a separate sensor as will be discussed further herein), the water mass can be found from Equation (2), which can be written with Equation (4), as:

$$m_w = \frac{1}{c_w \left( R_{th}\left(T_{hp}, T_w, A_{hp}, R_c, \dots\right) C_{hp} \frac{d^2 T_{hp}}{dt^2} + \frac{dT_{hp}}{dt}\left(1 + \alpha_{hp_{loss}} \cdot R_{th}\left(T_{hp}, T_w, A_{hp}, R_c, \dots\right)\right) \right)} \left( P_{el} - C_{hp} \frac{dT_{hp}}{dt} - \alpha_{hp_{loss}} \right.$$

$$\left. \cdot \left(T_{hp} - T_{atm}\right) - \alpha_{w_{loss}} \cdot \left(T_w - T_{atm}\right) - h(Q, m_w) \cdot A_s(m_w) \cdot \left(T_w - T_{hum_{in}}\right) - \dot{m}_w \cdot L_h \right)$$

(Eq. 6)

Equation (6) can also be simplified (i.e., when the second derivative of $T_{hp}$ is small) to:

$$m_w = \frac{1}{c_w \frac{dT_{hp}}{dt}} \left( P_{el} - C_{hp} \frac{dT_{hp}}{dt} - \alpha_{hp_{loss}} \cdot \left(T_{hp} - T_{atm}\right) - \alpha_{w_{loss}} \cdot \left(T_w - T_{atm}\right) - h(Q, m_w) \cdot A_s(m_w) \right.$$

$$\left. \cdot \left(T_w - T_{hum_{in}}\right) - \dot{m}_w \cdot L_h \right)$$

(Eq. 7)

It was found that Equation (7) can also be further simplified to neglect the initial water evaporation (i.e., the term $(\dot{m}_w \cdot L_h)$) to:

$$m_w = \frac{1}{c_w \frac{dT_{hp}}{dt}} \left( P_{el} - C_{hp} \frac{dT_{hp}}{dt} - \alpha_{hp_{loss}} \cdot \left(T_{hp} - T_{atm}\right) - \alpha_{w_{loss}} \cdot \left(T_w - T_{atm}\right) - h(Q, m_w) \cdot A_s(m_w) \right.$$

$$\left. \cdot \left(T_w - T_{hum_{in}}\right) \right)$$

(Eq. 8)

All parameters in Equation (8) can be determined from (i) the heater plate temperature, (ii) the estimated water bath temperature, (iii) the system parameters and (iv) known device parameters.

In addition, the accuracy of the water mass determination improves considerably when an average heater plate temperature gradient is introduced, during the period of investigation (i.e., time interval $t_b$ to $t_e$). In particular, the time

$t_b$ is determined where the first derivative in the heater plate gradient is in the range of 1.5-10 and more preferably in the range of 3 to 5 times the quasi-steady state heater plate temperature gradient. The time $t_e$ (or end time of the interval) is determined as a moment which is a similar time away from the quasi-steady state heater plate gradient time $t_s$ as the beginning time $t_b$, which fulfils the expression of $t_e \approx 2t_s - t_b$. In other words, the time distance between $t_e$ and $t_s$ is equal to the time distance between $t_s$ and $t_b$. The average heater plate temperature gradient is then determined according to the following expression.

$$\frac{1}{t_e - t_b} \int_{t_b}^{t_e} \frac{\mathrm{d}T_{hp}}{\mathrm{d}t} \, \mathrm{d}t$$

(Eq. 9)

Note that it is also possible to determine the initial water mass at multiple times and the average can be taken to calculate the water mass. Here the water mass can be calculated by changing both $t_b$ and $t_e$ (but still fulfilling the requirement on the gradient in heater plate temperature to calculate $t_b$), while a change in $t_s$ is also possible (still fulfilling requirements on the calculation of $t_s$). The advantage is that the water mass can be calculated fairly quickly (approximately 3-7 minutes, or 5 minutes, after power to the heater plate is switched ON). In addition, it is important to realize that Equation (8) is independent of the thermal resistance $R_{th}$ of the heater plate, which is a significant advantage.

Note that it is possible to calculate, with the known water temperature (i.e., whether predicted or measured via a separate temperature sensor as discussed herein), the evaporation rate during the unsteady phase (Fig. 5), which makes it possible to accurately predict the remaining water mass as a function of time. It appears that the water mass at any given time t follows from

$$m(t) \approx m_0 - \dot{m} \cdot t + m_{offset}(Pow)$$

Here $m_0$ is the initial water mass as determined according to both methods, $\dot{m}$ is the steady state evaporation rate which can be calculated with the transfer function as discussed herein, and $m_{offset}$ is a small offset, which depends on the power. In particular, $m_{offset}$ increases with power.

With this information, (i) an estimation of the total time to evaporate the water contained in the water tank can be made and a warning signal given to the humidifier user, as well as (ii) adapt the power setting to the heater plate to ensure that there is sufficient water in the water tank to last during the complete sleeping period (or duration of the humidification session or treatment period (e.g., during the night as the user sleeps)).

Another method for determining the water mass is to make use of the fact that after the initial steep rise of the heater plate temperature $T_{hp}$, the difference between heater plate temperature $T_{hp}$ and water temperature $T_w$ is almost constant, as shown in Fig. 7. Fig. 7 is a plot 146 of temperature difference 148 between heater plate and water for the run of the DOE of Fig. 5 according to an embodiment of the present disclosure. In that case, the temperature of the water mass can be very well predicted by:

$$T_w(t) = \frac{a}{b} - \left(\frac{a}{b} - T_w(0)\right) e^{-\frac{b}{m_w c_w}(t - t_0)}$$

(Eq. 10)

In Equation (9), the constants a and b follow from Equations (2) and (3). Note that $a/b = T_{w,s}$ the steady state water temperature. A similar relaxation time $\dfrac{m_w c_w}{b}$ can be used for the heater plate temperature $T_{hp}$ where the relaxation time starts at a similar time as for the first method, i.e., the time where the first derivative in the heater plate temperature is less than 50% larger than the quasi-steady state first derivative of the heater plate, and more preferably less than 10% larger than the quasi-steady state first derivative. This time is called $t_s$.

A simple function for the heater plate temperature $T_{hp}$ can be written as:

$$T_{hp}(t) = T_{hp,steady} - \left(T_{hp,steady} - T_{hp}(t_1)\right) e^{-\frac{b}{m_w c_w}(t-t_1)}$$

(Eq. 11)

From the steady state model, we know upfront (i.e., in advance) an approximate value of the steady state temperature of the heater plate $T_{hp,steady}$ which can even be verified experimentally, or an estimate can be found when the thermal resistance $R_{th}$ is used from a previous time the humidifier appliance has been used. This thermal resistance $R_{th}$ follows from Equation (1), which in steady state can be written as:

$$R_{th} = \frac{T_{hp,s} - T_{w,s}}{P_{el} - \alpha_{hp}(T_{hp} - T_a)}$$

(Eq. 12)

Note that it is also possible to warn a user or customer when this thermal resistance $R_{th}$ has become too large (i.e., greater than a given threshold thermal resistance), indicating that the metal plate on the water tank may be calcified or deposited debris.

Now the water mass can be obtained from Equation (11), when we plot $\ln\left(\frac{T_{hp,s} - T_{hp}(t_1)}{T_{hp,s} - T_{hp}(t)}\right)$ as a function of $t-t_1$ this will be a straight curve where the gradient will be $b/(m_w c_w)$, with $b$ given as:

$$b = \alpha_{w_{loss}} + h(Q) \cdot A_w + L_h \left(\frac{\dot{m}_w(T_{w,s}) - \dot{m}_w(T_w(0))}{T_{w,s} - T_w(0)}\right)$$

(Eq. 13)

A simplification of Equation (13) was found to be easier to use, which is given by:

$$b = \alpha_{w_{loss}} + h(Q) \cdot A_w + L_h \left(\frac{\dot{m}_w(T_{w,s})}{T_{w,s} - T_w(0)}\right)$$

(Eq. 14)

Here $T_{w,s}$ is the water temperature at steady state, which is preferably obtained from the steady state transfer function. Two options are considered to determine the steady state heater plate temperature. A first option would be via experiments; however, the disadvantage would be that it takes a long time. Another option is that the steady state heater plate temperature can be obtained according to:

$$T_{hp,s} \approx T_{w,s} + \left(T_{hp}(t_1) - T_{hp}(t_0) - \gamma\right)$$

(Eq. 15)

Here the steady water plate temperature can be calculated with the transfer function as discussed in co-pending patent application, US Serial No. 63/357,311, filed June 30, 2022 (Attorney Docket 2022PF00428), incorporated herein by reference in its entirety. The time $t_1$ is the time where the heater plate gradient is almost constant, or the second derivative almost zero, while $t_0$ is the time when the power is switched on, $\gamma$ is a small temperature correction, to correct for the slight change in difference in water and heater plate temperature over time. The parameter $\gamma$ is for all experiments in the range of 3-12°C, depending on power and initial water mass. The value of $\gamma$ increases with increasing power and decreasing initial water mass. A combination is also possible, i.e., first estimation of steady state heater plate temperature via option 1 and then verify with option 2.

This method, as discussed in the immediately preceding paragraphs with reference to Equation 11, is also independent of the thermal resistance of the metal plate of the water tank, which is a significant advantage. Furthermore, this method does not require an initial waiting period to apply power to the heater plate subsequent to turning the humidifier appliance ON.

In a further embodiment, the methods discussed herein can be combined to an advantage. That is, a first method, according to Equation (8), gives a faster result but the second method, according to solution of Equation (11), is an interesting alternative.

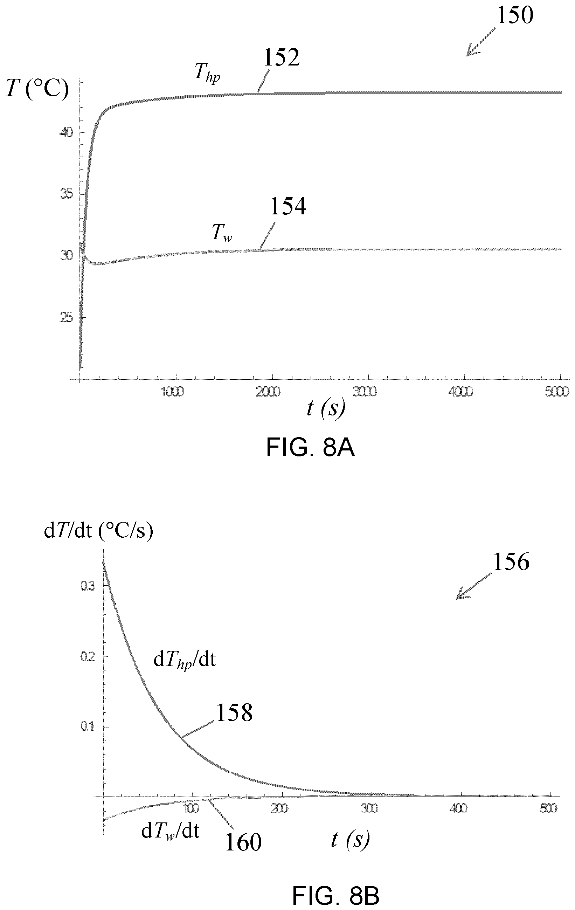

In case the initial water temperature is close to steady state water temperature, it may be difficult for the method, according to Equation (7), to measure the water mass. Preferably the initial water temperature is at least 5 degrees lower than the steady state water temperature (which can be estimated from the corresponding transfer function). It can be easily observed that when the initial water temperature is close to the steady state water temperature, the derivative of the heater plate temperature becomes close to zero very quickly. This is for instance, as shown in Figs. 8A and 8B, where a 10 °C larger water temperature is used, which is close to the steady state water temperature. In such an instance, a signal can be given, via a suitable signal output device, to the consumer that the initial water temperature is too large to determine the water mass accurately. In particular, Fig. 8 is a simulation of the run of the DOE of Fig. 5, but now with a 10 °C larger water temperature, where Fig. 8A is a temperature profile 150 of heater plate temperature 152 and water bath temperature 154 for time to reach equilibrium, and b) a plot 156 of temperature derivatives 158 and 160, respectively, for the heater plate and water bath.

In another embodiment, the humidifier further comprises a separate thermocouple or temperature sensor for detecting the temperature of the water bath. When an extra thermocouple is used in the water bath, the method for determining water mass is in principle the same as without the separate temperature sensor. In particular, the water mass can be determined with a similar procedure as the second method discussed herein above, but now with the heater plate temperature replaced by the water bath temperature (i.e., according to the solution of Equation (11)). Note that the water temperature can also be used to reduce or omit the waiting time before the constant electrical input power to the heater plate is switched on. The water temperature can also be used for the improved method 1 as here the water temperature is required at t= $t_s$. Further it is interesting to look at the time where the gradients in heater plate temperature and water temperature is similar, or the difference between the heater plate and water bath temperature remains constant.

In another embodiment, the first method, according to the solution of Equation (8), can be used initially at a larger power to the heater plate for a given initial duration and switched to steady state power for the heater plate after an estimate of the water mass has been obtained. That is, power control is provided to the heater plate, via the power control algorithm, that includes an accelerated steady state function for use in obtaining a steady state equilibrium faster than without the accelerated steady state function. The controller, via the power control algorithm, is configured to (i) initially set a power input to the heater plate to an increased power input compared to a quasi-steady state value, and (ii) responsive to a detection of at least one of the generated sensor output signals attaining at least a predetermined percentage of its quasi-steady state value, switch to quasi-steady state power control algorithm power level corresponding to quasi-steady state settings. A substantially faster time to equilibrium is possible with this method according to an embodiment of the present disclosure. An example of power control with maximum power for the given Run of DOE, has been calculated numerically wherein the first 300 seconds, a maximum power of 40 W is applied and then switched back to 20 W. It is thus possible to substantially decrease the time to steady state (i.e., from approximately 3000 seconds to 1000 seconds).

The first derivative, as discussed herein, is determined with standard techniques preferably based on a central method, i.e., data points before and after the moment when it is desired to determine the first derivative. It is also possible to smoothen the first derivative in the interesting time period via standard procedures, e.g., an exponential fitting function.

Although only a few exemplary embodiments have been described in detail above, those skilled in the art will readily appreciate that many modifications are possible in the exemplary embodiments without materially departing from the novel teachings and advantages of the embodiments of the present disclosure. Accordingly, all such modifications are intended to be included within the scope of the embodiments of the present disclosure as defined in the following claims. In the claims, means-plus-function clauses are intended to cover the structures described herein as performing the recited function and not only structural equivalents, but also equivalent structures.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa. One or more of the embodiments may be implemented by means of hardware comprising several distinct elements, and/or by means of a suitably programmed computer. In a device claim enumerating several means, several of these means may be embodied by one and the same item of hardware. The mere fact that certain measures are recited in mutually different dependent claims does not indicate that a combination of these measures cannot be used to an advantage.

**Claims**

1.  A humidifier (52) of a heated pass-over type for use in a sleep or respiratory therapy device that includes a blower section (56) having a blower for supplying a pressurized flow of breathable gas, comprising:

    a heater plate (70);
    a water reservoir (62) structured to house a volume of water (66), the water reservoir having a breathable gas inlet (72) and a humidified breathable gas outlet (74), wherein the water reservoir includes at least one surface (68) for contacting the heater plate;
    one or more sensors (86) configured to generate output signals conveying information about an operating status of the humidifier and ambient conditions; and
    a controller (88) configured to:

    (i) predict a water mass of the volume of water contained within the water reservoir at a predetermined time during a humidifier treatment session of a given treatment duration based on predetermined heat and water mass transfer functions of a given design of the humidifier, the output signals conveying information about the operating status of the humidifier and ambient conditions, and on a constant electrical input power to the heater plate;
    (ii) determine a length of time needed to evaporate, at a desired evaporation rate, all water of the predicted water mass; and
    (iii) at least one of (a) output a warning signal in response to the determined length of time to evaporate all water of the predicted water mass being insufficient for a remainder of the given treatment duration starting from the predetermined time, and (b) adapt a heater plate constant electrical input power setting to change the desired evaporation rate to a new evaporation rate of a current predicted water mass to ensure that the current predicted water mass is sufficient to last for a remainder of the given treatment duration and prevent the water reservoir from running dry prior to an ending of the given treatment duration of the humidifier treatment session.

2.  The humidifier (52) according to claim 1, wherein the predetermined time comprises (i) an initial time period at a start of the humidifier treatment session, or (ii) an intermediate time period of the humidifier treatment session, subsequent to the initial time period, or (iii) a combination of the initial time period and the intermediate time period.

3.  The humidifier (52) according to claim 2, wherein the initial time period comprises within a time period immediately after switching the humidifier power ON, but not yet applying the constant electrical input power to the heater plate.

4.  The humidifier (52) according to claim 1, wherein the controller (88) is configured to predict the water mass of the volume of water further according to a power control algorithm for humidifying the flow of breathable gas received at the breathable gas inlet of the water reservoir into a flow of humidified breathable gas at the humidified breathable gas outlet of the water reservoir, wherein the power control algorithm includes a transfer function in which a required power input to the heater plate is a function of a desired evaporation rate based upon generated sensor output signals.

5.  The humidifier (52) according to claim 1, wherein determining the water mass comprises determining a value of water mass multiple times over an initial water mass determination period of time, and calculating an initial water mass by averaging the multiple determined values of water mass obtained over the water mass determination period.

6.  The humidifier (52) according to claim 5, wherein the operating status of the humidifier includes a heater plate temperature, and wherein the initial water mass determination period of time comprises a period of time after constant electrical input power is applied to the heater plate up until a time $t_s$, wherein the time $t_s$ corresponds to a time when a condition that (i) a first derivative in the heater plate temperature is less than 50% larger than a quasi-steady state first derivative of the heater plate temperature or (ii) the first derivative in the heater plate temperature is less than 10% larger than the quasi-steady state first derivative of the heater plate temperature, has been reached.

7.  The humidifier (52) according to claim 1, wherein determining the length of time needed to evaporate all water of the predicted water mass is based on the predicted water mass and an average heater plate temperature gradient during the predetermined time.

8.  The humidifier (52) according to claim 1, wherein the one or more sensors (86) comprise a temperature sensor for measuring a temperature of the heater plate, but no separate temperature sensor for the measuring a temperature of the water (66) in the water reservoir (64), and

wherein predicting the water mass comprises predicting an initial water mass, wherein predicting the initial water mass comprises:

determining an initial water temperature based on the heater plate temperature during a delay period of heater plate power application which occurs immediately subsequent to switching of the humidifier ON but prior to an application of constant electrical input power to the heater plate,

measuring and recording the heater plate temperature during the delay period, wherein the delay period expires in response to a first derivative of the heater plate temperature with respect to time during the delay period having obtained a value less than or equal to a minimum first derivative threshold value,

applying, upon expiration of the delay period, the constant electrical input power to the heater plate and both (i) measuring and recording the heater plate temperature and (ii) determining at least another first derivative of the heater plate temperature with respect to time,

determining a time, based on the measured and recorded heater plate temperature over time, at which (i) the another first derivative in the heater plate temperature is less than 50% larger than a quasi-steady state first derivative of the heater plate temperature or (ii) the another first derivative in the heater plate temperature is less than 10% larger than the quasi-steady state first derivative of the heater plate temperature, has been reached,

creating a heater plate temperature profile that includes sampled heater plate temperatures collected over a profile creation period of time (i) beginning with the application of the constant electrical input power to the heater plate and (ii) ending upon detection of a variation in first derivative of the heater plate temperature to time determined during the profile creation period becoming less than or equal to a minimum first derivative variation threshold amount,

calculating a gradient in temperature of the water within the water reservoir based on the heater plate temperature profile,

estimating the water temperature based on (i) the initial determined water temperature, (ii) the calculated gradient in temperature of the water, and (iii) a total duration of time that the constant power to the heater plate has been applied, adjusted by a correction factor, and

predicting the initial water mass according to a water mass temperature relationship between (i) the heater plate temperature, (ii) the estimated water temperature, (iii) system parameters for the given humidifier design, and (iv) known humidifier device parameters.

9. The humidifier (52) according to claim 8, wherein the system parameters and humidifier device parameters include water-air interface surface area, heater plate heat loss coefficient, water heat loss coefficient, mass transfer coefficient, heater plate electrical input power, and heat transfer from blower to air.

10. The humidifier (52) according to claim 8, wherein the correction factor is determined based on (i) a change in heater plate temperature in which the heater plate temperature has increased in a range between 3 to 6 degrees from a temperature of the heater plate when the constant electrical input power to the heater plate is first applied to the heater plate subsequent the heater plate power application delay period, or (ii) an increase depending on the constant electrical input power and the predicted water mass.

11. The humidifier (52) according to claim 1, wherein the one or more sensors (86) comprise a temperature sensor for measuring a temperature of the heater plate, and a separate temperature sensor for the measuring a temperature of the water in the water reservoir, and

wherein the controller (88) is configured to predict the water mass of the volume of water based on the predetermined heat and water mass transfer functions that include heat and water mass temperature relationships between (i) the heater plate temperature, (ii) the estimated water temperature, (iii) system parameters for the given humidifier design, and (iv) known humidifier device parameters.

12. The humidifier (52) according to claim 11, wherein the system parameters and humidifier device parameters include water-air interface surface area, heater plate heat loss coefficient, water heat loss coefficient, mass transfer coefficient, heater plate electrical input power, and heat transfer from blower to air.

13. A gas delivery system (50) for delivering a pressurized flow of humidified breathable gas to a patient via a patient circuit, comprising:

a blower assembly (56) having a blower (58) adapted to generate the pressurized flow of breathable gas, and

EP 4 438 089 A1

a gas flow path including an inlet and an outlet; and
a humidifier (52) according to claim 1, wherein the humidifier is fluidically coupled between the blower and the patient circuit (80), wherein the blower assembly further includes at least one of the sensors (86) configured to generate output signals conveying information used in predicting the water mass.

14. A method for determining water mass in a humidifier of a heated pass-over type for use in humidifying a flow of breathable gas in a sleep or respiratory therapy device having a blower section that includes a blower for supplying a pressurized flow of breathable gas, the method comprising:

providing a heater plate;
providing a water reservoir structured to house a volume of water, the water reservoir having a breathable gas inlet and a humidified breathable gas outlet, wherein the water reservoir includes at least one surface for contacting the heater plate;
providing one or more sensors configured to generate output signals conveying information about an operating status of the humidifier and ambient conditions;
predicting, via a controller, a water mass of the volume of water contained within the water reservoir at a predetermined time during a humidifier treatment session of a given treatment duration based on predetermined heat and water mass transfer functions of a given design of the humidifier, the output signals conveying information about the operating status of the humidifier and ambient conditions, and on a constant electrical input power to the heater plate;
determining, via the controller, a length of time needed to evaporate, at a desired evaporation rate, all water of the predicted water mass; and
controlling, via the controller, at least one of (a) outputting a warning signal in response to the determined length of time to evaporate all water of the predicted water mass being insufficient for a remainder of the given treatment duration starting from the predetermined time, and (b) adapting a heater plate constant electrical input power setting to change the desired evaporation rate to a new evaporation rate of a current predicted water mass to ensure that the current predicted water mass is sufficient to last for a remainder of the given treatment duration and prevent the water reservoir from running dry prior to an ending of the given treatment duration of the humidifier treatment session.

15. The method of claim 14, wherein the one or more sensors comprise a temperature sensor for measuring a temperature of the heater plate, but no separate temperature sensor for the measuring a temperature of the water in the water reservoir, and
wherein predicting, via the controller, the water mass further comprises predicting an initial water mass, wherein predicting the initial water mass comprises:

determining an initial water temperature based on the heater plate temperature during a delay period of heater plate power application which occurs immediately subsequent to switching of the humidifier ON but prior to an application of constant electrical input power to the heater plate,
measuring and recording the heater plate temperature during the delay period, wherein the delay period expires in response to a first derivative of the heater plate temperature with respect to time during the delay period having obtained a value less than or equal to a minimum first derivative threshold value,
applying, upon expiration of the delay period, the constant electrical input power to the heater plate and both (i) measuring and recording the heater plate temperature and (ii) determining at least another first derivative of the heater plate temperature with respect to time,
determining a time, based on the measured and recorded heater plate temperature over time, at which (i) the another first derivative in the heater plate temperature is less than 50% larger than a quasi-steady state first derivative of the heater plate temperature or (ii) the another first derivative in the heater plate temperature is less than 10% larger than the quasi-steady state first derivative of the heater plate temperature, has been reached,
creating a heater plate temperature profile that includes sampled heater plate temperatures collected over a profile creation period of time (i) beginning with the application of the constant electrical input power to the heater plate and (ii) ending upon detection of a variation in first derivative of the heater plate temperature to time determined during the profile creation period becoming less than or equal to a minimum first derivative variation threshold amount,
calculating a gradient in temperature of the water within the water reservoir based on the heater plate temperature profile,
estimating the water temperature based on (i) the initial determined water temperature, (ii) the calculated gradient

18

in temperature of the water, and (iii) a total duration of time that the constant power to the heater plate has been applied, adjusted by a correction factor, and

predicting the initial water mass according to a water mass temperature relationship between (i) the heater plate temperature, (ii) the estimated water temperature, (iii) system parameters for the given humidifier design, and (iv) known humidifier device parameters.

36 34

10

32

TO
PATIENT

30

12
HUMIDIFIER
SECTION

24

BLOWER
SECTION

FROM
BLOWER

WATER TANK

22 WATER

POSITIVE AIRWAY PRESSURE SYSTEM

26

20 18 28 16

14

FIG. 1

FIG. 2

100

102   104   106   108

| System parameters | Description | Value | Units |
|---|---|---|---|
| $A_s$ | Water-air interface surface area | 0.0125 | [m2] |
| $\alpha_{hp_{loss}}$ | Heater plate heat loss coefficient | 0.140030 | [W/K] |
| $\alpha_{w_{loss}}$ | Water heat loss coefficient | 0.142785 | [W/K] |
| $k(Q)$ | Mass transfer coefficient | $0.0039397 + 16.161 \cdot Q$ | [m/s] |
| $h(Q)$ | Heat transfer coefficient | $3.7424 + 14872 \cdot Q$ | [W/(m2*K)] |
| $V_{hp}$ | Heater plate coil voltage | 11.7 | [V] |
| $R_{hp}(T_{hp})$ | Heater plate coil resistance | $3.0778 + 0.0021 \cdot (T_{hp} - 273.15)$ | [Ω] |
| $f(\Delta p, Q)$ | Heat transfer from blower to air | $1974.95 \cdot Q + 0.0020533 \cdot \Delta p + 1.6391 \cdot Q \cdot \Delta p$ | [W] |

FIG. 3

FIG. 4A

FIG. 4B

FIG. 5

FIG. 6A

FIG. 6B

FIG. 6C

$T_{hp} - T_w$

146

148

$T\,(^\circ\mathrm{C})$

$t\,(\mathrm{s})$

FIG. 7

FIG. 8A

FIG. 8B

Europäisches
Patentamt

European
Patent Office

Office européen
des brevets

# EUROPEAN SEARCH REPORT

**Application Number**

EP 23 16 5169

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 10 220 177 B2 (FISHER & PAYKEL HEALTHCARE LTD [NZ]) 5 March 2019 (2019-03-05) * the whole document, and especially the abstract, the claims and the figures * ----- | 1-15 | INV. A61M16/00 A61M16/10 A61M16/16 |
| A | US 2015/014874 A1 (WINSKI JEFFREY RONALD [US]) 15 January 2015 (2015-01-15) * the whole document, and especially the abstract, the claims and the figures * ----- | 1-15 | |
| A | WO 2014/102660 A1 (KONINKL PHILIPS NV [NL]) 3 July 2014 (2014-07-03) * the whole document, and especially the abstract, the claims and the figures * ----- | 1-15 | |
| A | US 2016/339200 A1 (BATH ANDREW RODERICK [AU] ET AL) 24 November 2016 (2016-11-24) * the whole document, and especially the abstract, the claims and the figures * ----- | 1-15 | |
| A | WO 2021/250573 A1 (RESMED SENSOR TECH LTD [IE]) 16 December 2021 (2021-12-16) * the whole document, and especially the abstract, the claims and the figures * ----- | 1-15 | TECHNICAL FIELDS SEARCHED (IPC) A61M |
| A | US 2019/262560 A1 (LIU PO-YEN [NZ] ET AL) 29 August 2019 (2019-08-29) * the whole document, and especially the abstract, the claims and the figures * ----- | 1-15 | |

The present search report has been drawn up for all claims

| Place of search | Date of completion of the search | Examiner |
|---|---|---|
| Munich | 14 September 2023 | Azaïzia, Mourad |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 16 5169

This annex lists the patent family members relating to the patent documents cited in the above-mentioned European search report.
The members are as contained in the European Patent Office EDP file on
The European Patent Office is in no way liable for these particulars which are merely given for the purpose of information.

14-09-2023

| Patent document cited in search report | | Publication date | Patent family member(s) | | Publication date |
|---|---|---|---|---|---|
| US 10220177 | B2 | 05-03-2019 | AU | 2010335072 A1 | 12-07-2012 |
| | | | CA | 2784852 A1 | 30-06-2011 |
| | | | CN | 102740918 A | 17-10-2012 |
| | | | CN | 104888330 A | 09-09-2015 |
| | | | DE | 112010005006 T5 | 15-11-2012 |
| | | | ES | 2398816 A1 | 21-03-2013 |
| | | | GB | 2509044 A | 25-06-2014 |
| | | | US | 2013174841 A1 | 11-07-2013 |
| | | | US | 2016375217 A1 | 29-12-2016 |
| | | | US | 2019217044 A1 | 18-07-2019 |
| | | | US | 2022040439 A1 | 10-02-2022 |
| | | | WO | 2011078706 A2 | 30-06-2011 |
| US 2015014874 | A1 | 15-01-2015 | CN | 104144723 A | 12-11-2014 |
| | | | EP | 2819727 A1 | 07-01-2015 |
| | | | JP | 2015511851 A | 23-04-2015 |
| | | | RU | 2014139594 A | 20-04-2016 |
| | | | US | 2015014874 A1 | 15-01-2015 |
| | | | WO | 2013128347 A1 | 06-09-2013 |
| WO 2014102660 | A1 | 03-07-2014 | BR | 112015015298 A2 | 11-07-2017 |
| | | | CN | 104884112 A | 02-09-2015 |
| | | | EP | 2938378 A1 | 04-11-2015 |
| | | | JP | 6416111 B2 | 31-10-2018 |
| | | | JP | 2016501661 A | 21-01-2016 |
| | | | RU | 2015131016 A | 03-02-2017 |
| | | | US | 2015306335 A1 | 29-10-2015 |
| | | | WO | 2014102660 A1 | 03-07-2014 |
| US 2016339200 | A1 | 24-11-2016 | NZ | 629531 A | 29-04-2016 |
| | | | NZ | 717147 A | 25-08-2017 |
| | | | TW | 201517943 A | 16-05-2015 |
| | | | US | 2016339200 A1 | 24-11-2016 |
| | | | US | 2020338299 A1 | 29-10-2020 |
| | | | WO | 2015058255 A1 | 30-04-2015 |
| WO 2021250573 | A1 | 16-12-2021 | NONE | | |
| US 2019262560 | A1 | 29-08-2019 | AU | 2017297977 A1 | 21-02-2019 |
| | | | AU | 2023203071 A1 | 22-06-2023 |
| | | | CN | 109789288 A | 21-05-2019 |
| | | | CN | 115253002 A | 01-11-2022 |
| | | | EP | 3487573 A1 | 29-05-2019 |
| | | | JP | 7123032 B2 | 22-08-2022 |
| | | | JP | 2019520951 A | 25-07-2019 |
| | | | JP | 2022119922 A | 17-08-2022 |

EPO FORM P0459

For more details about this annex : see Official Journal of the European Patent Office, No. 12/82

page 1 of 2

EP 4 438 089 A1

## ANNEX TO THE EUROPEAN SEARCH REPORT
## ON EUROPEAN PATENT APPLICATION NO.

EP 23 16 5169

This annex lists the patent family members relating to the patent documents cited in the above-mentioned European search report.
The members are as contained in the European Patent Office EDP file on
The European Patent Office is in no way liable for these particulars which are merely given for the purpose of information.

14-09-2023

| Patent document cited in search report | Publication date | Patent family member(s) | Publication date |
|---|---|---|---|
| | | SG 10202100532P A | 25-02-2021 |
| | | SG 11201900520Y A | 27-02-2019 |
| | | US 2019262560 A1 | 29-08-2019 |
| | | US 2023016953 A1 | 19-01-2023 |
| | | WO 2018016974 A1 | 25-01-2018 |

EPO FORM P0459

For more details about this annex : see Official Journal of the European Patent Office, No. 12/82

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10220177 B

- US 63357311